# EUROPEAN PATENT SPECIFICATION

(11) **EP 0 438 849 B1**
(45) Date of publication and mention of the grant of the patent: **23.11.1994**
(21) Application number: 90300810.0
(22) Date of filing: 25.01.1990
(51) Int. Cl.: C10M 133/56

(54) **Dicarboxylic acid derivatives of succinimides or succinamides useful in dispersant compositions**
Dicarbonsäure-Derivate von Bernsteinsäureimiden oder -amiden, die in Dispergierzusammensetzungen verwendar sind
Succinimides ou succinamides modifiés par un acide dicarboxylique utiles dans des compositions dispersantes

(43) Date of publication of application: 31.07.1991
(73) Proprietor: ETHYL PETROLEUM ADDITIVES LIMITED, Bracknell, Berkshire RG12 2UW (GB)
(72) Inventor: Scattergood, Roger, Reading, Berkshire RG12 2UW (GB); Walters, David Kenvyn, Camberley, Surrey, GU17 7TR (GB)
(74) Representative: Collier, Jeremy Austin Grey

(56) References cited:
- WO-A-87/03003
- GB-A- 1 065 595
- GB-A- 2 140 811
- US-A- 3 374 174
- US-A- 4 663 064
- US-A- 4 686 054

## Description

This invention relates to lubricant dispersants and to compositions containing them. More particularly, this invention relates to aliphatic succinimides and aliphatic succinimide-containing compositions of enhanced performance capabilities.

A continuing problem in the art of lubrication is to provide lubricant compositions which satisfy the demands imposed upon them by the original equipment manufacturers. One such requirement is that the lubricant not contribute to premature deterioration of seals, clutch face plates or other parts made from fluoroelastomers. Unfortunately, and as is well known, basic nitrogen-containing dispersants such as succinimide dispersants commonly used in oils tend to exhibit a strong adverse effect upon fluoroelastomers, by causing them to lose their flexibility and tensile strength, to become embrittled, and in severe cases, to disintegrate. It has been postulated that the co-presence of zinc-containing additives such as zinc dialkyldithiophosphates tends to increase the severity of this problem. Contemporary test methods for evaluating fluoroelastomer compatibility of lubricant compositions are the Volkswagen P.VW 3334 Seal Test and the CCMC Viton Seal Test (CEL L-39-T-87 Oil/Elastomer Compatibility Test).

U.S. Pat. No. 4,548,724 describes the formation of dispersant additives by reacting an organic polycarboxylic acid having 3-6 carboxyl groups in the molecule with a polyalkenyl succinimide of a polyamine. It is taught in the patent that Viton elastomer deterioration is inhibited when this reaction is carried out so that at least about 30 atom %, preferably 50-100 atom %, more preferably 85-100 atom %, say 100 atom %, of the reactive nitrogen atoms in the succinimide have been reacted to form an amine with the carboxyl groups of the acid or mixture of acids.

US-A-4,663-064 discloses a dispersant to improve the compatibility of lubricating oil compositions with elastomer seals prepared by coupling partially glycolated succinimides with an organic diacid.

A new effective, practical way of overcoming the antagonism of conventional succinimide and similar basic nitrogen-containing dispersants toward fluoroelastomers would be a welcome contribution to the art.

According to the present invention there is provided a process which comprises (i) reacting at least one polyamine with at least one acyclic hydrocarbyl substituted succinic acylating agent in which the substituent contains an average of at least 40 carbon atoms to form a product having at least some non-acylated nitrogen atoms in the molecule, and (ii) reacting the product so formed without any intermediate reaction with a dicarboxylic acid acylating agent in which the two carboxyl groups are separated from each other by at least 3 aliphatic carbon atoms, the process being characterised in that in step (i) the acylating agent is reacted with the polyamine in a mole ratio of from 2.00 to 2.85 moles of acylating agent per mole of polyamine, and in that in step (ii) the dicarboxylic acid acylating agent is employed in an amount such that the mole ratio of such acylating agent is from 0.10 to 2.50 moles per mole of said polyamine with the proviso that the total mole ratio of the acylating agents in (i) and (ii) per mole of said polyamine is in the range of 2.40 to 5.00.

In a preferred embodiment said polyamine is a polyalkylene polyamine;
said hydrocarbyl substituent of said hydrocarbyl substituted succinic acid is polyisobutenyl; and
said dicarboxylic acid is one in which the two carboxyl groups are separated from each other by at least four aliphatic carbon atoms.

This process provides an acyclic hydrocarbyl substituted succinimide, acyclic hydrocarbyl succinic acid, acyclic hydrocarbyl substituted succinamide, and/or ester/amide formed with polyamine, which has been reacted with a dicarboxylic acid in which the two carboxyl groups are separated from each other by at least 3, and preferably by at least 4, aliphatic carbon atoms. The resultant product causes significantly less degradation of fluoroelastomers than the corresponding untreated succinimide, succinamide, or mixed ester/amide. For convenience, the acyclic hydrocarbyl substituted succinimides, hydrocarbyl substituted succinamides and mixed ester/amides of hydrocarbyl-substituted succinic acid formed with polyamines are collectively referred to as "basic nitrogen dispersants".

Other embodiments of this invention involve the provision of lubricant compositions and lubricant additive concentrates containing a basic nitrogen dispersant which has been reacted with a dicarboxylic acid of the type just described.

Typical dicarboxylic acids which can be employed in the practice of this invention include glutaric, adipic, pimelic, suberic, azeleic, sebacic, 1,12-dodecanedioic, brassylic, 1,6-hex-3-enedioic, traumatic (1,12-dodec-2-enedioic), dimerized unsaturated fatty acids such as dimerized linoleic acid, and like acids in which the two carboxyl groups are separated from each other by at least three and preferably by at least four aliphatic carbon atoms. Most preferably the carboxyl groups are on terminal carbon atoms, however this is not essential. Ordinarily, the dicarboxylic acid will contain from 5 up to 40 carbon atoms in the molecule. Preferably the dicarboxylic acid will contain from 5 to 12 carbon atoms in the molecule. Mixtures of two or more of the foregoing dicarboxylic acid acylating agents can be used.

Another embodiment of this invention is the provision of a dispersant prepared as above having the ability when formulated in a finished engine lubricating oil of satisfying the requirements of the CCMC Viton Seal Test (CEC L-39-T-87 Oil/Elastomer Compatibility Test).

Pursuant to still further embodiments of this invention there are provided lubricating oil additive concentrates and lubricating oil compositions as just described which additionally contain a zinc-containing additive complement, especially one or a mixture of zinc dihydrocarbyldithiophosphates, such as one or a combination of zinc dialkyldithiophosphates, one or a combination of zinc diaryldithiophosphates, or a combination of one or more zinc dialkyldithiophosphates with one or more zinc diaryldithiophosphates.

These and other embodiments and features of this invention will be apparent from the ensuing description and appended claims.

Acyclic hydrocarbyl-substituted succinic acid acylating agents and methods for their preparation are well known to those skilled in the art and are extensively reported in the patent literature. See, for example the following U.S. Patents:

| | | |
|---|---|---|
| 3,018,247 | 3,231,587 | 3,399,141 |
| 3,018,250 | 3,272,746 | 3,401,118 |
| 3,018,291 | 3,287,271 | 3,513,093 |
| 3,172,892 | 3,311,558 | 3,576,743 |
| 3,184,474 | 3,331,776 | 3,578,422 |
| 3,185,704 | 3,341,542 | 3,658,494 |
| 3,194,812 | 3,346,354 | 3,658,495 |
| 3,194,814 | 3,347,645 | 3,912,764 |
| 3,202,678 | 3,361,673 | 4,110,349 |
| 3,215,707 | 3,373,111 | 4,234,435 |
| 3,219,666 | 3,381,022 | |

As indicated in such prior patents, the acyclic hydrocarbyl substituted succinic acylating agents include the hydrocarbyl-substituted succinic acids, the hydrocarbyl-substituted succinic anhydrides, the hydrocarbyl-substituted succinic acid halides (especially the acid fluorides and acid chlorides), and the esters of the hydrocarbyl-substituted succinic acids and lower alcohols (e.g., those containing up to about 7 carbon atoms), that is, hydrocarbylsubstituted compounds which can function as carboxylic acylating agents. Of these compounds, the hydrocarbylsubstituted succinic acids and the hydrocarbyl-substituted succinic anhydrides and mixtures of such acids and anhydrides are generally preferred, the hydrocarbyl-substituted succinic anhydrides being particularly preferred.

Preferably, the hydrocarbyl substituent of the succinic acylating agent used in step (i) is an alkyl or, more preferably, an alkenyl group containing 20 or more carbon atoms. Particularly preferred acylating agents for use in (i) have alkenyl substituents with a number average molecular weight (as determined by gel permeation chromatography) of at least 560 (and more preferably in the range of 900 to 10,000, most preferably in the range of 980 to 5,000), especially where the alkenyl substituents are formed from polyolefins made from C₃ or C₄ olefins (e.g., isobutylene, 1-butene, and mixtures of butenes containing the same as the predominant components). Polyisobutenylsuccinic acids, polyisobutenyl succinic anhydrides, and mixtures of polyisobutenylsuccinic acids and polyisobutenylsuccinic anhydrides are most especially preferred for use in the practice of step (i) above.

Suitable polyamines for use in step (i) above are described in many of the above cited U.S. patents. For best results, the polyamines should contain at least two primary amino groups in the molecule.

The preferred polyamines used in the practice of this invention are the alkylene polyamines represented by the formula

H₂N(CH₂)ₙ(NH(CH₂)ₙ)ₘNH₂

wherein n is 2 to 10 (preferably 2 to 4, more preferably 2 to 3, and most preferably 2) and m is 1 to 10, (preferably 1 to 6). Illustrative are diethylene triamine, triethylene tetramine, tetraethylene pentamine, spermine, and pentaethylene hexamine. Preferred for use is tetraethylene pentamine or a mixture of ethylene polyamines which approximates tetraethylene pentamine such as "DOW E-100" (a commercial mixture available from Dow Chemical Company, Midland, Michigan).

In the practice of this invention, the dicarboxylic acid acylating agent (i.e., the reactant in step (ii) above) is reacted with a succinimide, succinamide, or mixed succinic ester/amide dispersant formed from a polyamine and which has non-acylated nitrogen atoms in the molecule.

Of the several types of basic nitrogen dispersants which may be subjected to reaction with a dicarboxylic acid having at least three and preferably at least four aliphatic carbon atoms between the carboxyl groups, it is preferred to utilize a succinimide dispersant formed with a polyamine, particularly one or more polyalkylene polyamines.

As used herein the term "succinimide" is meant to encompass the completed reaction product and is intended to encompass compounds wherein the product may have amide, amidine, and/or salt linkages in addition to the imide linkage of the type that results from the reaction of a primary amino group and an anhydride moiety.

The reactions involved in steps (i) and (ii) are conducted at conventional temperatures in the range of 80°C to 200°C, more preferably 140°C to 180°C. These reactions may be conducted in the presence or absence of an ancillary diluent or liquid reaction medium, such as a mineral lubricating oil solvent. If the reaction is conducted in the absence of an ancillary solvent of this type, such is usually added to the reaction product on completion of the reaction. In this way the final product is in the form of a convenient solution in lubricating oil and thus is compatible with a lubricating oil base stock. Suitable solvent oils are the same as the oils used as a lubricating oil base stock and these generally include lubricating oils having a viscosity (ASTM D 445) of 2 to 40, preferably 3 to 12 mm²/sec at 100°C, with the primarily paraffinic mineral oils such as Solvent 100 Neutral being particularly preferred. Other types of lubricating oil base stocks can be used, such as synthetic lubricants including polyesters, poly-α-olefins, and the like. Blends of mineral oil and synthetic lubricating oils are also suitable for various applications in accordance with this invention.

Finished lubricating oil compositions of this invention are prepared containing the dispersant of this invention together with conventional amounts of other additives to provide their normal attendant functions. Thus use may be made of such conventional additives as viscosity index improvers, dispersant viscosity index improvers, rust inhibitors, metal detergent additives, antioxidants, antiwear additives, extreme pressure additives, and the like. Reference may be had to the various U.S. Patents referred to hereinabove for exemplary disclosures of various conventionally used additives for lubricating oils.

A particularly preferred ancillary additive used in the lubricant compositions is a grafted copolymer dispersant VI improver of the type described in U.S. Pat. No. 4,519,929.

As noted above, there are two accepted contemporary test procedures for determining the extent to which a basic nitrogen dispersant causes degradation of a fluoroelastomer. One of these, the Volkswagen P.VW 3334 Seal Test involves keeping a test specimen of fluoroelastomer (VITON AK6) in an oil blend at 150°C for 96 hours and then comparing both the change in elongation to break and the tensile strength of the test specimen to the corresponding properties of a fresh specimen of the same fluoroelastomer. The exposed test specimen is also examined for the presence of cracks. In these tests, a lubricant passes the test if the exposed test specimen exhibits a change in elongation to break (as compared to an untested specimen) of no more than -25% and a tensile strength (as compared to an untested specimen) of no more than -20%, and possesses no cracks. Another test which can be used to measure the effect of lubricant additives on fluoroelastomers is the CCMC Viton Seal Test, CEC L-39-T-87 Oil/Elastomer Compatibility Test. This test is similar to the VW Test except that it is a 7-day test rather than a 4-day test, the elastomer is VITON RE I, and the pass/fail points are -50% tensile strength and -60% elongation. Experiments conducted to date indicate that the CCMC Seal Test is less stringent than the VW Seal Test.

The practice and the benefits achievable by the practice of this invention are illustrated in the following specific examples which are not to be construed as limitations on this invention.

### EXAMPLE 1

In a first stage reaction, polyisobutenylsuccinic anhydride (PIBSA) formed from polyisobutylene (number average molecular weight = 1300) and tetraethylene pentamine (TEPA) in a mole ratio of 2.05:1 are reacted at 165-170°C for 4 hours. In a second stage reaction, adipic acid is added to the first stage reaction product in amount equivalent to 1 mole per mole of TEPA used in the first stage and the resultant mixture is heated at 165-170°C for 1.5 hours. The succinimide is thus formed using a total mole ratio of anhydrides to TEPA of 3.05:1. To provide a handleable concentrate, the reaction product is suitably diluted with 100 solvent neutral mineral oil such that the nitrogen content of the blend is about 1.8%.

### EXAMPLE 2

The procedure of Example 1 is repeated except that in the second stage, azelaic acid is used in amount equivalent to a mole ratio of 1:1 relative to the TEPA used in the first stage. Thus the total mole ratio of anhydrides to polyamine is again 3.05:1.

### EXAMPLE 3

Using the same general procedure as in Examples 1 and 2, the following dicarboxylic acids are individually employed in equivalent quantities in place of adipic acid and azelaic acid: pimelic acid, suberic acid, 1,12-dodecanedioic acid, brassylic acid, and traumatic acid.

### COMPARATIVE EXAMPLE A

The procedure of Example 1 is repeated except that an equivalent amount of acetic acid is used in step (ii) rather than adipic acid.

### COMPARATIVE EXAMPLE B

The procedure of Example 1 is repeated except that an equivalent amount of octanoic acid is used in step (ii) rather than adipic acid.

### COMPARATIVE EXAMPLE C

The procedure of Example 1 is repeated except that an equivalent amount of phthalic acid is used in step (ii) rather than adipic acid.

In order to determine the compatibility of various succinimide dispersants with fluoroelastomers, a series of finished crankcase lubricating oils for use in internal combustion engines containing various substituted succinimide dispersants were formulated. Each such oil contained, in addition to the succinimide dispersant, conventional amounts of overbased sulfonates, zinc dialkyl dithiophosphate, antioxidant, viscosity index improver, rust inhibitor, and antifoam agent to provide an SAE 15W/40 crankcase lubricant oil. Each such lubricant contained an amount of the succinimide dispersant to provide a nitrogen content of 0.13%.

The resultant finished lubricating oils were subjected to the CCMC Viton Seal Test, CEC L-39-T-87 Oil/Elastomer Compatibility Test. The results of this series of tests are summarized in the following Table.

**Table**

| Results of CCMC Viton Seal Tests | | | |
|---|---|---|---|
| Test No. | Succinimide Used | Change in Elongation to Break Compared to Fresh Seal, % | Tensile Strength Compared to Fresh Seal, % |
| 1 | Example 1 | -42.3 | -12.8 |
| 2 | Example 2 | -40.2 | - 6.8 |
| 3 | Example A | -58.5 | -38.9 |
| 4 | Example B | -47.5 | -39.2 |
| 5 | Example C | -49.1 | -26.2 |
| 6 | Control* | -49.9 | -31.2 |

| | | | |
|---|---|---|---|
| * Formulation containing commercial succinimide dispersant | | | |

The dispersants utilized according to the invention can be incorporated in a wide variety of lubricants. They can be used in lubricating oil compositions, such as automotive crankcase lubricating oils, automatic transmission fluids, and gear oils in effective amounts to provide active ingredient concentrations in finished formulations generally within the range of 0.5 to 10 weight percent, for example, 1 to 9 weight percent, preferably 2 to 8 weight percent, of the total composition. Conventionally, the dispersants are admixed with the lubricating oils as dispersant solution concentrates which usually contain up to about 50 weight percent of the active ingredient additive compound dissolved in mineral oil, preferably a mineral oil having an ASTM D-445 viscosity of 2 to 40, preferably 3 to 12 centistokes at 100°C. The lubricating oil includes not only hydrocarbon oils of lubricating viscosity derived from petroleum but also include natural oils such as rapeseed oil and synthetic lubricating oils such as hydrogenated polyolefin oils; hydrogenated and unhydrogenated α-olefin oligomers, dimers and/or trimers; alkyl esters of dicarboxylic acids, complex esters of dicarboxylic acid, polyglycol and alcohol; alkyl esters of carbonic or phosphoric acids; polysilicones; fluorohydrocarbon oils; and mixtures or lubricating oils and synthetic oils in any proportion. The term "lubricating oil" for this disclosure includes all the foregoing. The useful dispersant may be conveniently dispersed as a concentrate of 10 to 80 weight percent of mineral oil, e.g., Solvent 100 Neutral oil with or without other additives being present and such concentrates are a further embodiment of this invention.

As noted above, such lubricating oils compositions containing the dispersants of the present invention will also contain other well-known additives such as the zinc dialkyl (C₃-C₈) dithiophosphate wear inhibitors, generally present in amounts of 0.5 to 5 weight percent. Useful detergents include the oil-soluble normal basic or over-based metal, e.g., calcium, magnesium, and barium salts of petroleum naphthenic acids, petroleum sulfonic acids, alkyl benzene sulfonic acids, oil-soluble fatty acids, alkyl salicylic acids, sulfurized or unsulfurized alkyl phenates, and hydrolyzed or unhydrolyzed phosphosulfurized polyolefins. Gasoline engine crankcase lubricants typically contain, for example, from 0.5 to 5 weight percent of one or more detergent additives. Diesel engine crankcase oils may contain substantially higher levels of detergent additives. Preferred detergents are the calcium and magnesium normal or overbased phenates, sulfurized phenates or sulfonates.

Oxidation inhibitors include hindered phenols (e.g., 2,6-di-tert-butyl-para-cresol, 2,6-di-tert-butyl-phenol, 4,4'-methylenebis(2,6-di-tert-butylphenol), and mixed methylene bridged polyalkyl phenols, amines, sulfurized phenols and alkyl phenothiazines usually present in amounts of from 0.001 to 1 weight percent.

Pour point depressants which may be present in amounts of from 0.01 to 1 weight percent include wax alkylated aromatic hydrocarbons, olefin polymers and copolymers, acrylate and methacrylate polymers and copolymers.

Viscosity index improvers, the concentrations of which may vary from 0.2 to 15 weight percent, (preferably from 0.5 to 5 weight percent) depending on the viscosity grade required, include hydrocarbon polymers grafted with, for example, nitrogen-containing monomers, olefin polymers such as polybutene, ethylene-propylene copolymers, hydrogenated polymers and copolymers and terpolymers of styrene with isoprene and/or butadiene, polymers of alkyl acrylates or alkyl methacrylates, copolymers of alkyl methacrylates with N-vinyl pyrrolidone or dimethylaminoalkyl methacrylate, post-grafted polymers of ethylene-propylene with an active monomer such as maleic anhydride which may be further reacted with an alcohol or an alkylene polyamine, and styrene/maleic anhydride polymers post-treated with alcohols and amines.

Antiwear activity can be provided by 0.01 to 2 weight percent of the aforementioned metal dihydrocarbyl dithiophosphates and the corresponding precursor esters, phosphosulfurized pinenes, sulfurized olefins and hydrocarbons, sulfurized fatty esters and alkyl polysulfides. Preferred are the zinc dihydrocarbyl dithiophosphates which are salts of dihydrocarbyl esters of dithiophosphoric acids.

Other additives include effective amounts of friction modifiers or fuel economy additives such as the alkyl phosphonates as disclosed in U.S. 4,356,097, aliphatic hydrocarbyl substituted succinimides as disclosed in EPO 0020037, dimer acid esters, as disclosed in U.S. 4,105,571, oleimide, which are present in the oil in amounts of 0.1 to 5 weight percent. Glycerol oleates are another example of fuel economy additives and these are usually present in very small amounts, such as 0.05 to 0.2 weight percent based on the weight of the formulated oil.

## Claims

1. A process which comprises (i) reacting at least one polyamine with at least one acyclic hydrocarbyl substituted succinic acylating agent in which the substituent contains an average of at least 40 carbon atoms to form a product having non-acylated nitrogen atoms in the molecule, and (ii) reacting the product so formed without any intermediate reaction with a dicarboxylic acid acylating agent in which the two carboxyl groups are separated from each other by at least 3 aliphatic carbon atoms, the process being characterised in that in step (i) the acylating agent is reacted with the polyamine in a mole ratio of from 2.00 to 2.85 moles of acylating agent per mole of polyamine, and in that in step (ii) the dicarboxylic acid acylating agent is employed in an amount such that the mole ratio of such acylating agent is from 0.10 to 2.50 moles per mole of said polyamine with the proviso that the total mole ratio of the acylating agents in (i) and (ii) per mole of said polyamine is in the range of 2.40 to 5.00.

2. A process according to claim 1 wherein said polyamine is an alkylene polyamine of the formula
H₂N(CH₂)ₙ (NH(CH₂)ₙ)ₘNH₂
wherein n is 2 to 10 and m is 1 to 10.

3. A process according to claim 2 wherein said polyamine is tetraethylene pentamine or a mixture of ethylene polyamines which approximates tetraethylene pentamine.

4. A process according to claim 1, 2 or 3 wherein said hydrocarbyl substituted succinic acylating agent has an alkenyl substituent having a number average molecular weight (as determined by gel permeation chromatography) of from 900 to 10,000.

5. A process according to any preceding claim wherein said dicarboxylic acid is one in which the two carboxyl groups are separated from each other by at least four aliphatic carbon atoms.

6. A process according to any preceding claim wherein:
said polyamine is a polyalkylene polyamine;
said hydrocarbyl substituent of said hydrocarbyl substituted succinic acylating agent is polyisobutenyl; and
said dicarboxylic acid is one in which the two carboxyl groups are separated from each other by at least four aliphatic carbon atoms.

7. A dispersant obtainable by a process according to any one of claims 1 to 6.

8. A lubricating oil additive concentrate containing a dispersant according to claim 7.

9. A lubricating oil composition containing a major amount of lubricating oil and a minor amount of a dispersant in accordance with claim 7.

10. A lubricating oil composition according to claim 9 further comprising a minor amount of one or more zinc dihydrocarbyl dithiophosphates.

## Patentansprüche

1. Verfahren, bei welchem man
(i) ein Polyamin mit mindestens einer mit einem acyclischen Kohlenwasserstoff substituierten Bernsteinsäure als Acylierungsmittel, wobei der Substituent im Durchschnitt mindestens 40 Kohlenstoffatome enthält, zur Bildung eines Produktes mit nichtacylierten Stickstoffatomen im Molekül umsetzt und
(ii) das so erhaltene Produkt ohne Zwischenstufen mit einer Dicarbonsäure als Acylierungsmittel umsetzt, in welcher beide Carboxylgruppen durch mindestens drei Kohlenstoffatome voneinander getrennt sind,
wobei das Verfahren dadurch gekennzeichnet ist, daß man
im ersten Schritt (i) das Acylierungsmittel mit dem Polyamin in einem Molverhältnis von 2,00 bis 2,85 Mol Acylierungsmittel pro Mol Polyamin umsetzt und
im zweiten Schritt (ii) die Dicarbonsäure als Acylierungsmittel in einer so bemessenen Menge einsetzt, daß das Molverhältnis dieses Acylierungsmittels 0,10 bis 2,50 Mol pro Mol des Polyamins beträgt, mit der Maßgabe, daß das Molverhältnis der Acylierungsmittel in den Schritten (i) und (ii) insgesamt in einem Bereich von 2,40 bis 5,00 pro Mol Polyamin liegt.

2. Verfahren gemäß Anspruch 1, bei dem das Polyamin ein Alkylenpolyamin der Formel ist:
H₂N(CH₂)ₙ(NH(CH₂)ₙ)ₘNH₂ ,
in der n 2 bis 10 und m 1 bis 10 bedeuten.

3. Verfahren gemäß Anspruch 2, bei dem das Polyamin Tetraethylenpentamin oder ein Gemisch aus Ethylenpolyaminen ist, welches Tetraethylenpentamin nahekommt.

4. Verfahren gemäß einem der Ansprüche 1, 2 oder 3, bei dem die mit Kohlenwasserstoff substituierte Bernsteinsäure als Acylierungsmittel einen Alkenylsubstituenten mit einem zahlenmittleren Molekulargewicht von 900 bis 10000 aufweist (bestimmt mittels Gelpermeationschromatographie).

5. Verfahren gemäß einem der vorstehenden Ansprüche, bei dem die Dicarbonsäure eine Dicarbonsäure darstellt, deren beide Carboxylgruppen durch mindestens vier aliphatische Kohlenstoffatome voneinander getrennt sind.

6. Verfahren gemäß einem der vorstehenden Ansprüche, bei dem
- das Polyamin ein Polyalkylenpolyamin,
- der Kohlenwasserstoffsubstituent der mit einem Kohlenwasserstoff substituiertne Bernsteinsäure als Acylierungsmittel Polyisobutenyl und
- die Dicarbonsäure eine Dicarbonsäure darstellt, deren beide Carboxylgruppen durch mindestens vier aliphatische Kohlenstoffatome voneinander getrennt sind.

7. Dispergiermittel, welches mit Hilfe eines Verfahrens gemäß einem der vorstehenden Ansprüche 1 bis 6 erhältlich ist.

8. Schmieröladditivkonzentrat, enthaltend ein Dispergiermittel gemäß Anspruch 7.

9. Schmierölzubereitung, enthaltend eine Hauptmenge an Schmieröl und eine kleinere Menge an Dispergiermittel gemäß Anspruch 7.

10. Schmierölzubereitung gemäß Anspruch 9, welche zusätzlich eine kleinere Menge eines oder mehrerer Zinkdihydrocarbyldithiophosphate enthält.

## Revendications

1. Procédé qui comprend (i) la réaction d'au moins une polyamine avec au moins un agent acylant succinique à substituant hydrocarbyle acyclique dans lequel le substituant contient un nombre moyen d'au moins 40 atomes de carbone pour former un produit ayant des atomes d'azote non acylés dans la molécule, et (ii) la réaction du produit ainsi formé, sans aucune réaction intermédiaire, avec un agent d'acylation du type acide dicarboxylique dans lequel les deux groupes carboxyle sont séparés l'un de l'autre par au moins trois atomes de carbone aliphatiques, le procédé étant caractérisé en ce que, dans l'étape (i), l'agent d'acylation est amené à réagir avec la polyamine en un rapport molaire de 2,00 à 2,85 moles d'agent d'acylation par mole de polyamine, et en ce que, dans l'étape (ii), l'agent d'acylation du type acide dicarboxylique est utilisé en une quantité telle que le rapport molaire de cet agent d'acylation à la polyamine soit un rapport de 0,10 à 2,50 moles par mole de ladite polyamine, sous réserve que le rapport molaire total des agents d'acylation dans les étapes (i) et (ii) à ladite polyamine soit compris dans l'intervalle de 2,40 à 5,00 par mole de ladite polyamine.

2. Procédé suivant la revendication 1, dans lequel la polyamine est une alkylènepolyamine de formule
H₂N(CH₂)ₙ (NH(CH₂)ₙ)ₘNH₂
dans laquelle n a une valeur de 2 à 10 et m a une valeur de 1 à 10.

3. Procédé suivant la revendication 2, dans lequel la polyamine est la tétraéthylènepentamine ou un mélange d'éthylènepolyamines dont la composition est proche de la tétraéthylènepentamine.

4. Procédé suivant la revendication 1, 2 ou 3, dans lequel l'agent acylant succinique à substituant hydrocarbyle possède un substituant alcényle ayant une moyenne numérique du poids moléculaire (telle qu'elle est déterminée par chromatographie de perméation sur gel) de 900 à 10 000.

5. Procédé suivant l'une quelconque des revendications précédentes, dans lequel l'acide dicarboxylique est un acide dans lequel les deux groupes carboxyle sont séparés l'un de l'autre par au moins quatre atomes de carbone aliphatiques.

6. Procédé suivant l'une quelconque des revendications précédentes, dans lequel :
la polyamine est une polyalkylène-polyamine ;
le substituant hydrocarbyle de l'agent acylant succinique à substituant hydrocarbyle est un substituant polyisobutényle ; et
l'acide dicarboxylique est un acide dans lequel les deux groupes carboxyle sont séparés l'un de l'autre par au moins quatre atomes de carbone aliphatiques.

7. Dispersant pouvant être obtenu par un procédé suivant l'une quelconque des revendications 1 à 6.

8. Concentré d'additifs pour huile lubrifiante, contenant un dispersant suivant la revendication 7.

9. Composition d'huile lubrifiante contenant une quantité dominante d'une huile lubrifiante et une quantité secondaire d'un dispersant suivant la revendication 7.

10. Composition d'huile lubrifiante suivant la revendication 9, comprenant en outre une petite quantité d'un ou plusieurs dihydrocarbyldithiophosphates de zinc.
